# EUROPEAN PATENT SPECIFICATION

(11) **EP 2 813 228 B1**
(45) Date of publication and mention of the grant of the patent: **08.12.2021**
(21) Application number: 14165935.9
(22) Date of filing: 25.04.2014
(51) Int. Cl.: A61K 31/519, A61P 17/00

(54) **AGENT FOR IMPROVEMENT OF SKIN BARRIER FUNCTION**
MITTEL ZUR VERBESSERUNG DER FUNKTION DER HAUTBARRIERE
AGENT POUR AMÉLIORER LA FONCTION DE BARRIÈRE CUTANÉE

(30) Priority: 25.04.2013 JP 2013092378
(43) Date of publication of application: 17.12.2014
(73) Proprietor: Japan Tobacco Inc., Tokyo 105-6927 (JP); Kyoto University, Kyoto-shi, Kyoto 606-8501 (JP)
(72) Inventor: Tanimoto, Atsuo, Takatsuki, Osaka 569-1125 (JP); Ueda, Yoshifumi, Takatsuki, Osaka 569-1125 (JP); Kimoto, Yukari, Takatsuki, Osaka 569-1125 (JP); Amano, Wataru, Takatsuki, Osaka 569-1125 (JP); Kabashima, Kenji, Kyoto-shi, Kyoto 606-8501 (JP)
(74) Representative: Vossius & Partner Patentanwälte Rechtsanwälte mbB

(56) References cited:
- EP-A1- 0 715 852
- WO-A1-2007/032624
- WO-A1-2010/020905
- WO-A1-2011/097087
- JP-A- 2009 256 269
- US-A1- 2007 135 461
- US-A1- 2008 312 259
- US-A1- 2011 136 778
- FUJII YASUTOMO ET AL: "Effects of the Janus Kinase Inhibitor CP-690550 (Tofacitinib) in a Rat Model of Oxazolone-Induced Chronic Dermatitis", PHARMACOLOGY (BASEL), vol. 91, no. 3-4, 11 March 2013 (2013-03-11), pages 207-213, XP009180746,
- JORDAN S FRIDMAN ET AL: "Preclinical Evaluation of Local JAK1 and JAK2 Inhibition in Cutaneous Inflammation", JOURNAL OF INVESTIGATIVE DERMATOLOGY, vol. 131, no. 9, 16 June 2011 (2011-06-16) , pages 1838-1844, XP055146781, ISSN: 0022-202X, DOI: 10.1038/jid.2011.140
- BYUNG-HAK KIM ET AL: "Kurarinone regulates immune responses through regulation of the JAK/STAT and TCR-mediated signaling pathways", BIOCHEMICAL PHARMACOLOGY, vol. 85, no. 8, 1 April 2013 (2013-04-01), pages 1134-1144, XP055146768, ISSN: 0006-2952, DOI: 10.1016/j.bcp.2013.01.005

## Description

### [TECHNICAL FIELD]

The present invention relates to a novel pharmaceutical use of a JAK inhibitor as defined in the appended claims. More specifically, the present invention relates to a skin barrier function improving agent containing a JAK inhibitor, and a therapeutic or preventive agent for skin diseases such as senile xerosis and asteatosis containing a JAK inhibitor.

### [BACKGROUND ART]

Janus kinase (JAK) belongs to cytoplasmic protein tyrosine kinase family including JAK1, JAK2, JAK3, and TYK2.

JAK inhibitors have been reported in Patent Literature 1, Patent Literature 2, Patent Literature 3, etc.

Skin plays an important role for maintenance of a living body through skin barrier function including the biological protection function against external stimuli such as physical impacts, temperature, exposure to ultraviolet rays or chemicals, or infection, and the moisturizing function that prevents water loss from inside a living body. The skin barrier function is exerted by the stratum corneum that is composed of several to several tens layers piled up regularly, and located in the outermost layer of the epidermis. It is known that deterioration in skin barrier function causes skin troubles such as dry skin disease and rough skin.

As skin barrier function-related proteins, filaggrin, loricrin, involucrin, β-defensin 3, etc., are known (Non-Patent Literature 1).

Filaggrin is produced as its precursor, profilaggrin, in a keratinocyte existing in the granular layer directly beneath the stratum corneum. In the course of the final differentiation of granular cells, profilaggrin is degraded to filaggrin as a monomer. Filaggrin changes the shape of a keratinocyte into a flat shape that is characteristic of a corneocyte of the stratum corneum by making keratin fibers aggregate inside the keratinocyte. Further, filaggrin is degraded to amino acids inside the stratum corneum and released. It has been reported that these amino acids have high water solubility and hygroscopicity, and are principal components constituting natural moisturizing factors (hereinafter, abbreviated as NMF) (Non-Patent Literature 1).

It is expected that increase of filaggrin protein production by increase of mRNA expression of profilaggrin in a keratinocyte will increase the quantity of amino acids as components of NMF in the stratum corneum, and essentially improve the moisturizing function of the stratum corneum.

Loricrin and involucrin are important proteins that form a cornified envelope (CE) coating beneath the cell membrane of corneocytes. Loricrin and involucrin are produced in the stratum spinosum to the granular layer in the course of differentiation of keratinocyte, and cross-linked to the cell membrane of keratinocyte by an enzyme, transglutaminase, to form CE as an insoluble cell membrane-like structure, and thus contribute to the stability of the cytoskeleton and structure of the corneocytes (Non-Patent Literature 1). However, when the production amounts of loricrin and involucrin are reduced due to various factors, formation of the cornified envelope (CE) is inadequate, and keratinization is not properly achieved. As a result, it is believed that the skin barrier function is deteriorated, and skin symptoms such as rough skin and dry skin are exhibited.

Antimicrobial peptides such as β-defensin 3 are closely involved in the first-line defense in the skin against invading pathogens combined with the physical barrier (Non-Patent Literature 2).

The condition that the skin dries and loses gloss to become rough due to a reduction in secretion of sebum and sweat is called xeroderma (asteatosis). Rice bran-like scales and shallow cracks arise to exhibit an ichthyosiform appearance, and the patient may complain slight itching. This is sometimes observed as one of the changes that occur with aging. Since the skin barrier function is deteriorated, the patient is susceptible to external stimuli (Non-Patent Literature 3).

Contact dermatitis refers to an eczematous inflammatory response developed as a result of contact between a foreign stimulant or an antigen (hapten) and the skin. It is believed that contact dermatitis is more likely to occur when the skin barrier function is deteriorated. Contact dermatitis is broadly classified into irritant contact dermatitis and allergic contact dermatitis (Non-Patent Literature 4).

Ichthyosis vulgaris is a disease of high prevalence characterized by desquamation and dry skin mainly on extensor surfaces of the extremities. Ichthyosis vulgaris is an inherited disorder of keratinization caused by a single gene, and it has been reported that ichthyosis vulgaris is caused by mutations in the gene encoding filaggrin, and that the degree of loss or reduction of filaggrin protein leads to varying the degree of impaired keratinization (Non-Patent Literature 5).

Netherton syndrome is a severe inherited skin disease associated with congenital ichthyosiform erythroderma or atopic dermatitis-like eruption, and caused by mutations in the serine protease inhibitor Kazal-type 5 (SPINK5) gene which encodes Lymphoepithelial Kazal-type-related inhibitor (LEKTI). Lack of LEKTI causes stratum corneum detachment secondary to epidermal protease hyperactivity. This defect of skin barrier function favors allergen absorption, and is believed to be an underlying cause for atopic dermatitis in a patient suffering from Netherton syndrome (Non-Patent Literature 6).

Peeling skin syndrome is a disease characterized by detachment of the epidermal stratum corneum. As peeling skin syndrome, type A as a non-inflammatory type and type B as an inflammatory type are known. The nonsense mutation in CDSN, which leads to complete loss of the function of the corneodesmosin, causes type B peeling skin syndrome (Non-Patent Literature 7).
Non-Patent Literature 13 reports on the effects of the Janus Kinase Inhibitor CP-690550 (Tofacitinib) in a rat model of oxazolone-induced chronic dermatitis.
Patent Literature 4 relates to salt forms of (R)-3-4-(4-(7H-pyrrolo[2,3-d]pyrimidin-4-yl)-1H-pyrazol-1-yl)-3-cyclo-pentylpropanenitrile that are useful in the modulation of Janus kinase activity.
Non-Patent Literature 14 reports on the preclinical evaluation of local JAK1 and JAK2 inhibition in cutaneous inflammation.
Non-Patent Literature 15 discloses that Kurarinone regulates immune responses through regulation of the JAK/STAT and TCR-mediated signaling pathways.
Patent Literature 5 (Patent family member of Patent Literature 3) relates to nitrogen-containing spirocyclic compounds and pharmaceutical uses thereof.
Patent Literatures 6 and 7 relate to pyrolo{2,3-d}pyrimidine compounds, their use as Janus kinase (JAK) inhibitors, pharmaceutical compositions containing these compounds, and methods for the preparation of these compounds.

Therefore, it is believed that an increase in expression of filaggrin, loricrin, involucrin, β-defensin 3, etc., improves the skin barrier function, and is effective for treating or preventing skin diseases such as rough skin and dry skin, for example, senile xerosis, asteatosis, eczema, contact dermatitis, ichthyosis vulgaris, Netherton syndrome and type B peeling skin syndrome.

### [CITATION LISTS]

### [PATENT LITERATURES]

[Patent Literature 1] WO 02/096909 A
[Patent Literature 2] US 2007/0135461 A
[Patent Literature 3] JP 2011-46700 A
[Patent Literature 4] US 2008/312259 A1
[Patent Literature 5] US 2011/136778 A1
[Patent Literature 6] WO 2010/020905 A1
[Patent Literature 7] WO 2011/097087 A1

### [NON-PATENT LITERATURES]

[Non-Patent Literature 1] CANDI, E et al. The cornified envelope: a model of cell death in the skin. Nat Rev Mol Cell Biol. Apr 2005, Vol.6, No.4, pages 328-340.
[Non-Patent Literature 2] GALLO, RL et al. Microbial symbiosis with the innate immune defense system of the skin. J Invest Dermatol. Oct 2011, Vol.131, No.10, pages 1974-1980.
[Non-Patent Literature 3] Hiroshi SHIMIZU "Atarashii hifukagaku dainihan (Textbook of Modern Dermatology, 2nd edition)", page 69, published in 2011.
[Non-Patent Literature 4] "Sesshokuhifuen sinryou gaidorain (Contact dermatitis medical guideline)", The Japanese Journal of Dermatology: 119(9), 1757-1793, 2009).
[Non-Patent Literature 5] SMITH, FJ et al. Loss-of-function mutations in the gene encoding filaggrin cause ichthyosis vulgaris. Nat Genet. Mar 2006, Vol.38, No.3, pages 337-342.
[Non-Patent Literature 6] BRIOT, A et al. Kallikrein 5 induces atopic dermatitis-like lesions through PAR2-mediated thymic stromal lymphopoietin expression in Netherton syndrome. J Exp Med. May 11 2009, Vol.206, No.5, pages 1135-1147.
[Non-Patent Literature 7] OJI, V et al. Loss of corneodesmosin leads to severe skin barrier defect, pruritus, and atopy: unraveling the peeling skin disease. Am J Hum Genet. Aug 13 2010, Vol.87, No.2, pages 274-281.
[Non-Patent Literature 8] SAVINI, I et al. Characterization of keratinocyte differentiation induced by ascorbic acid: protein kinase C involvement and vitamin C homeostasis. J Invest Dermatol. Feb 2002, Vol.118, No.2, pages 372-379.
[Non-Patent Literature 9] HOLZMANN, S et al. A model system using tape stripping for characterization of Langerhans cell-precursors in vivo. J Invest Dermatol. May 2004, Vol.122, No.5, pages 1165-1174.
[Non-Patent Literature 10] KAPLAN, DH et al. Early immune events in the induction of allergic contact dermatitis. Nat Rev Immunol. Feb 2012, Vol.12, No.2, pages 114-124.
[Non-Patent Literature 11] BELL, E et al. Production of a tissue-like structure by contraction of collagen lattices by human fibroblasts of different proliferative potential in vitro. Proc Natl Acad Sci U S A. Mar 1979, Vol.76, No.3, pages 1274-1278.
[Non-Patent Literature 12] MIYAMOTO, T et al. Itch-associated response induced by experimental dry skin in mice. Jpn J Pharmacol. Mar 2002, Vol.88, No.3, pages 285-292.
[Non-Patent Literature 13] Fuji Yasumoto et al: "Effects of the Janus Kinase Inhibitor CP-690550 (Tofacitinib) in a Rat Model of Oxazolone-Induced Chronic Dermatitis", Pharmacology (Basel), vol. 91, no. 3-4, 11 March 2013 (2013-03-11), pages 207-213.
[Non-Patent Literature 14] Jordan S Fridman et al: "Preclinical Evaluation of Local JAK1 and JAK2 Inhibition in Cutaneous Inflammation", Journal of Investigative dermatology, vol. 131, no. 9, 16 June 2011 (2011-06-16), pages 1838-1844, ISSN: 0022-202X, DOI: 10.1038/jid.2011.140.
[Non-Patent Literature 15] Byung-Hak Kim et al: "Kurarinone regulates immune responses through regulation of the JAK/STAT and TCR-mentioned signaling pathways", Biochemical pharmacology, vol. 85, no. 8, 1 April 2013 (2013-04-01), pages 1134-1144, ISSN: 0006-2952, DOI: 10.1016/j.bcp.2013.01.005.

### [SUMMARY OF THE INVENTION]

### [PROBLEM TO BE SOLVED BY THE INVENTION]

The problem to be solved by the invention is to provide a novel pharmaceutical use of a JAK inhibitor.

### [MEANS FOR SOLVING THE PROBLEM]

The present inventors have made diligent studies for developing a novel pharmaceutical use of a JAK inhibitor, and found for the first time that a JAK inhibitor increases the expression amounts of filaggrin, loricrin, involucrin and β―defensin 3 as skin barrier function-related proteins; that a JAK inhibitor significantly increases NMF production in a Tape Stripping-treated mouse; and that a JAK inhibitor significantly accelerates a reduction in the transepidermal water loss (hereinafter, referred to as TEWL) in a dry skin mouse model, namely improves the skin barrier function. As a result, the inventors have found for the first time that a JAK inhibitor is effective for various skin diseases caused by deterioration in the skin barrier function, and achieved the present invention.

Specifically, the present invention includes the following items:
1. An agent for use in the prevention or treatment of a skin disease selected from the group consisting of senile xerosis, asteatosis, ichthyosis vulgaris, Netherton syndrome and type B peeling skin syndrome, comprising a JAK inhibitor as an active ingredient, wherein the JAK inhibitor is a compound represented by the following chemical structural formula: 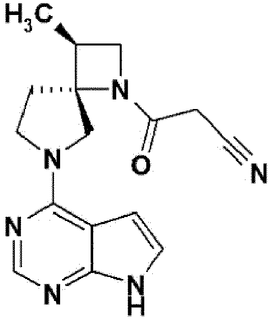 or a pharmaceutically acceptable salt thereof.
2. The agent for use according to item 1, wherein the skin disease selected in item 1 is senile xerosis.
3. The agent for use according to item 1, wherein the skin disease selected in item 1 is asteatosis.
4. The agent for use according to item 1, wherein the skin disease selected in item 1 is ichthyosis vulgaris.
5. The agent for use according to item 1, wherein the skin disease selected in item 1 is Netherton syndrome.
6. The agent for use according to item 1, wherein the skin disease selected in item 1 is type B peeling skin syndrome.

### [EFFECT OF THE INVENTION]

The present invention provides a novel pharmaceutical use of a JAK inhibitor as defined in the appended claims.

### [BRIEF DESCRIPTION OF THE DRAWINGS]

[Fig. 1] A chart illustrating an increase in profilaggrin mRNA amount by a JAK inhibitor in a newborn normal human epidermal keratinocyte. (Example 1)
[Fig. 2] A chart illustrating an increase in loricrin mRNA amount by the JAK inhibitor in a newborn normal human epidermal keratinocyte. (Example 1)
[Fig. 3] A chart illustrating an increase in involucrin mRNA amount by the JAK inhibitor in a newborn normal human epidermal keratinocyte. (Example 1)
[Fig. 4] A chart illustrating an increase in β-defensin 3 mRNA amount by the JAK inhibitor in a newborn normal human epidermal keratinocyte. (Example 1)
[Fig. 5] A chart illustrating NMF production increasing action by Compound A in a Tape Stripping-treated mouse.

### (Example 2)

[Fig. 6] A chart illustrating NMF production increasing action by monocitrate of Compound B (Reference Example) in the Tape Stripping-treated mouse. (Example 2)
[Fig. 7] A chart illustrating suppression of ear swelling by Compound A in a contact dermatitis model mouse. (Example 3)
[Fig. 8] A chart illustrating an increase in filaggrin and loricrin protein amounts by Compound A in the presence of IL-4/IL-13 in a three-dimensional culture skin model.

### (Example 4)

[Fig. 9] A chart illustrating an increase in filaggrin and loricrin protein amounts by Compound A in the absence of IL-4/IL-13 in a three-dimensional culture skin model.

### (Example 4)

[Fig. 10] A chart illustrating an increase in profilaggrin mRNA amount by Compound A in a dry skin model mouse.

### (Example 5)

[Fig. 11] A chart illustrating NMF production increasing action by Compound A in a dry skin model mouse. (Example 5)
[Fig. 12] A chart illustrating the action of accelerating a reduction in TEWL by Compound A in a dry skin model mouse.

### (Example 5)

### [MODE FOR CARRYING OUT THE INVENTION]

Terms and phrases used herein are defined as below.

A compound represented by the following chemical structural formula: 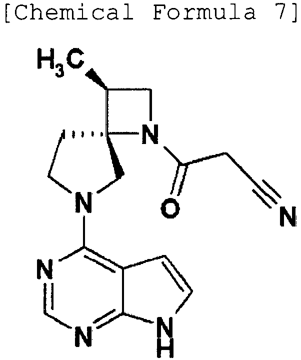 is called Compound A.

The chemical name of Compound A is 3-[(3*S*,4*R*)-3-methyl-6-(7*H*-pyrrolo[2,3-*d*]pyrimidin-4-yl)-1,6 -diazaspiro[3.4]octan-1-yl]-3-oxopropanenitrile.

The chemical name of Compound A is 3-[(3S,4R)-3-methyl-6-(7H-pyrrolo[2,3-d]pyrimidin-4-yl)-1,6 -diazaspiro[3.4]octan-1-yl]-3-oxopropanenitrile.

A compound represented by the following structural formula: 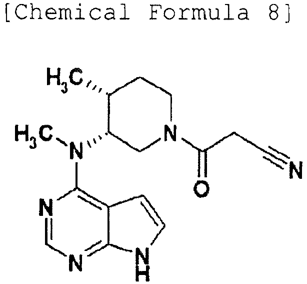 is called Compound B (Reference Example).

The chemical name of Compound B is 3-{(3*R*,4*R*)-4-methyl-3-[methyl(7*H*-pyrrolo[2,3-*d*]pyrimidin-4-yl)amino]piperidin-1-yl}-3-oxopropanenitrile.

The chemical name of Compound B is 3-{(3*R*,4*R*)-4-methyl-3-[methyl(7*H*-pyrrolo[2,3-*d*]pyrimidin-4-yl)amino]piperidin-1-yl}-3-oxopropanenitrile.

The following structural formula: 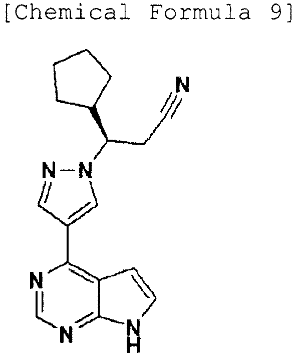 is called Compound C (Reference Example).

The chemical name of Compound C is (3*R*)-3-cyclopentyl-3-[4-(7*H*-pyrrolo[2,3-*d*]pyrimidin-4-yl)-1 *H*-pyrazol-1-yl]propanenitrile.

The chemical name of Compound C is (3*R*)-3-cyclopentyl-3-[4-(7*H*-pyrrolo[2,3-*d*]pyrimidin-4-yl)-1 *H*-pyrazol-1-yl]propanenitrile.

Compound A can be produced according to the method described in Preparation 6 of Patent Literature 3.

Compound B can be produced according to the method described in Patent Literature 1.

Compound C can be produced according to the method described in Patent Literature 2.

Compounds A, B and C are known to inhibit JAK.

The "pharmaceutically acceptable salt" may be any non-toxic salts of Compound A, B or C, and includes a salt with an inorganic acid, a salt with an organic acid, a salt with an inorganic base, a salt with an organic base, and a salt with an amino acid, etc.

The salt with an inorganic acid includes a salt with hydrochloric acid, nitric acid, sulfuric acid, phosphoric acid, hydrobromic acid, etc.

The salt with an organic acid includes a salt with oxalic acid, maleic acid, citric acid, fumaric acid, lactic acid, malic acid, succinic acid, tartaric acid, acetic acid, trifluoroacetic acid, gluconic acid, ascorbic acid, methanesulfonic acid, benzenesulfonic acid, p-toluenesulfonic acid, etc.

The salt with an inorganic base includes sodium salt, potassium salt, calcium salt, magnesium salt, ammonium salt, etc.

The salt with an organic base includes a salt with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine, etc.

The salt with an amino acid includes a salt with lysine, arginine, aspartic acid, glutamic acid, etc.

According to well-known methods, each salt may be obtained by reacting Compound A, B or C with an inorganic base, an organic base, an inorganic acid, an organic acid or an amino acid.

Compound A, B or C may be labeled with isotopes (e.g., ³H, ¹⁴C, ³⁵S, etc.).

Preferable Compound A, B or C, or a pharmaceutically acceptable salt thereof is substantially purified Compound A, B or C, or a pharmaceutically acceptable salt thereof. A more preferable one is Compound A, B or C, or a pharmaceutically acceptable salt thereof which is purified in the purity of 80% or more.

The "pharmaceutical composition" includes an oral preparation such as tablet, capsule, granule, powder, lozenge, syrup, emulsion and suspension, or a parenteral preparation such as external preparation, suppository, injection, eye drop, nasal drug and pulmonary drug.

The pharmaceutical composition of the present invention is produced by appropriately mixing Compound A, or a pharmaceutically acceptable salt thereof with at least one or more types of pharmaceutically acceptable carriers in appropriate amounts according to methods known in the technical field of medicinal preparation. The content of Compound A,
or a pharmaceutically acceptable salt thereof in the pharmaceutical composition depends on its dosage forms, dosage amounts, etc., and for example, is 0.1 to 100% by weight of the entire composition.

The "pharmaceutically acceptable carrier" includes various conventional organic or inorganic carrier substances for pharmaceutical materials, e.g., an excipient, a disintegrant, a binder, a fluidizer, and a lubricant for solid preparations, a solvent, a solubilizing agent, a suspending agent, a tonicity agent, a buffer, and a soothing agent for liquid preparations, and a base, an emulsifier, a humectant, a stabilizer, a stabilizing agent, a dispersant, a plasticizer, a pH regulator, an absorption promoter, a gelling agent, an antiseptic, a filler, a resolvent, a solubilizing agent, and a suspending agent for semisolid preparations. Further, an additive including a preserving agent, an antioxidant agent, a colorant, and a sweetening agent may be used, if needed.

The "excipient" includes lactose, white soft sugar, D-mannitol, D-sorbitol, cornstarch, dextrin, microcrystalline cellulose, crystalline cellulose, carmellose, carmellose calcium, sodium carboxymethyl starch, low-substituted hydroxypropyl cellulose, gum arabic, etc.

The "disintegrant" includes carmellose, carmellose calcium, carmellose sodium, sodium carboxymethyl starch, croscarmellose sodium, crospovidone, low-substituted hydroxypropyl cellulose, hydroxypropyl methylcellulose, crystalline cellulose, etc.

The "binder" includes hydroxypropyl cellulose, hydroxypropyl methylcellulose, povidone, crystalline cellulose, white soft sugar, dextrin, starch, gelatin, carmellose sodium, gum arabic, etc.

The "fluidizer" includes light anhydrous silicic acid, magnesium stearate, etc.

The "lubricant" includes magnesium stearate, calcium stearate, talc, etc.

The "solvent" includes purified water, ethanol, propylene glycol, macrogol, sesame oil, corn oil, olive oil, etc.

The "solubilizing agent" includes propylene glycol, D-mannitol, benzyl benzoate, ethanol, triethanolamine, sodium carbonate, sodium citrate, etc.

The "suspending agent" includes benzalkonium chloride, carmellose, hydroxypropyl cellulose, propylene glycol, povidone, methyl cellulose, glyceryl monostearate, etc.

The "tonicity agent" includes glucose, D-sorbitol, sodium chloride, D-mannitol, etc.

The "buffer" includes sodium hydrogen phosphate, sodium acetate, sodium carbonate, sodium citrate, etc.

The "soothing agent" includes benzyl alcohol, etc.

The "base" includes water, animal or plant oils (e.g., olive oil, corn oil, peanut oil, sesame oil, castor oil, etc.), lower alcohols (e.g., ethanol, propanol, propylene glycol, 1,3-butylene glycol, phenol, etc.), higher fatty acid and ester thereof, waxes, higher alcohol, polyhydric alcohol, hydrocarbons (e.g., white petrolatum, liquid paraffin, paraffin, etc.), hydrophilic petrolatum, purified lanolin, absorptive ointment, hydrous lanolin, hydrophilic ointment, starch, pullulan, gum arabic, tragacanth gum, gelatin, dextran, cellulose derivative (e.g., methyl cellulose, carboxymethyl cellulose, hydroxyethyl cellulose, hydroxypropyl cellulose, etc.), synthetic polymer (e.g., carboxyvinyl polymer, sodium polyacrylate, polyvinyl alcohol, polyvinyl pyrrolidone, etc.), propylene glycol, macrogol (e.g., macrogol 200-600, etc.), and combinations of two or more kinds of these.

The "preserving agent" includes ethyl parahydroxybenzoate, chlorobutanol, benzyl alcohol, sodium dehydroacetate, sorbic acid, etc.

The "antioxidant agent" includes sodium sulfite, ascorbic acid, etc.

The "colorant" includes food dye (e.g., Food Red No. 2 or No. 3, Food Yellow No. 4 or No. 5, etc.), β-carotene, etc.

The "sweetening agent" includes saccharin sodium, dipotassium glycyrrhizinate, aspartame, etc.

The pharmaceutical composition of the present invention may be orally or parenterally (e.g., locally, rectally, intravenously, etc.) administered to a mammal except a human being (e.g., a mouse, a rat, a hamster, a guinea pig, a rabbit, a cat, a dog, a pig, a cow, a horse, sheep, a monkey, etc.) as well as to a human being. A dosage amount depends on subjects, diseases, symptoms, dosage forms, administration routes, etc., and is usually, for example, in the range from about 0.01 mg to 1 g per day with comprising Compound A as an active ingredient. The dose may be administered at a time or in several divided doses.

An external preparation can be applied, for example, by application, inunction or spraying depending on the dosage form, etc. An application amount of the external preparation to the affected area can be selected depending on the content of the active ingredient, etc., and can be applied, for example, at a time or in several divided amounts per day.

The phrase "JAK" refers to one or two or more enzymes of JAK1, JAK2, JAK3, and TYK2 belonging to JAK family.

The phrase "inhibit JAK" refers to inhibiting functions of JAK to disappear or attenuate its activity, and inhibiting one or two or more enzymes belonging to JAK family.

The phrase "inhibit JAK" refers to preferably "inhibit human JAK". The inhibition of functions or the disappearance or attenuation of the activity is conducted preferably in the situations of human clinical application.

The "JAK inhibitor" may be any substances which inhibit JAK, and may be, for example, low-molecular weight compounds, nucleic acid, polypeptide, protein, antibody, vaccine, etc. The "JAK inhibitor" is preferably a "human JAK inhibitor".

The skin disease is preferably senile xerosis or asteatosis.

JAK inhibitors disclosed herein are Compounds A, B or C, or a pharmaceutically acceptable salt thereof. The JAK inhibitor for use according to the present invention is Compound A.

The term treatment used herein includes amelioration of a symptom, prevention of an aggravation, maintenance of a remission, prevention of an exacerbation, and prevention of a recurrence. The term prevention used herein refers to suppressing occurrence of a symptom.

An embodiment of the present invention includes a pharmaceutical composition for use in treating or preventing a skin disease selected from the group consisting of senile xerosis and asteatosis, comprising a JAK inhibitor as defined in the appended claims and a pharmaceutically acceptable carrier.

Herein disclosed is a skin barrier function improving method, characterized by administering to a mammal a pharmaceutically effective amount of a JAK inhibitor.

Herein disclosed is a method for increasing the transcription of a gene selected from the group consisting of filaggrin, loricrin, involucrin and β-defensin 3, characterized by administering to a mammal a pharmaceutically effective amount of a JAK inhibitor.

Herein disclosed is a method for increasing the production of a protein selected from the group consisting of filaggrin, loricrin, involucrin and β-defensin 3, characterized by administering to a mammal a pharmaceutically effective amount of a JAK inhibitor.

Herein disclosed is a method for increasing the production of NMF, characterized by administering to a mammal a pharmaceutically effective amount of a JAK inhibitor.

Herein disclosed is a method for reducing TEWL, characterized by administering to a mammal a pharmaceutically effective amount of a JAK inhibitor.

The mammal is a human being, a mouse, a rat, a hamster, a guinea pig, a rabbit, a cat, a dog, a pig, a cow, a horse, sheep, a monkey, etc., and is preferably a human being.

The human being is more preferably a person suffering from a disease who is in need of medical care.

An embodiment of the present invention includes the JAK inhibitor as defined in the appended claims for use in treating or preventing a skin disease selected from the group consisting of senile xerosis and asteatosis.

Herein disclosed is a use of a JAK inhibitor for improving skin barrier function.

Herein disclosed is a use of a JAK inhibitor for increasing the transcription of a gene or increasing the production of a protein selected from the group consisting of filaggrin, loricrin, involucrin and β-defensin 3.

The present invention is preferably a therapeutic or preventive agent for senile xerosis or asteatosis, comprising Compound A.

The present invention is preferably a pharmaceutical composition, comprising Compound A,
and a pharmaceutically acceptable carrier.

### [Example 1]

Experiment 1. Increase in mRNA amount of skin barrier-related protein by JAK inhibitor in newborn normal human epidermal keratinocyte

A newborn normal human epidermal keratinocyte {LONZA Ltd. } known to differentiate by calcium stimulation <Non-Patent Literature 8> was used in the experiment. Newborn normal human epidermal keratinocytes {LONZA Ltd.} were cultured in EpiLife^{®} Medium with 0.06 mM Calcium {Cascade Biologics, Inc.} supplemented with Human Keratinocyte Growth Supplement {Cascade Biologics, Inc.} according to the instruction manual of the cells. Newborn normal human epidermal keratinocytes were seeded in a 12-well plate at 5 × 10⁵ cells/well, and cultured at 37°C under 5% CO₂ for two hours. After removing the culture supernatant from the cells, a culture medium containing various concentrations of test substance and 1.3 mmol/L calcium chloride was added in the presence or absence of human IL-4 and human IL-13 {R&D Systems, Inc.} at a final concentration of 100 ng/mL, respectively, and the cells were cultured at 37°C under 5% CO₂ for five days. Compound A, monocitrate of Compound B or monophosphate of Compound C was used as the test substance.

Total RNA was purified from the above cultured cells using the GenElute Mammalian Total RNA Miniprep Kit {Sigma-Aldrich Corporation} according to the instruction manual.

The resultant RNA was reverse transcribed into cDNA by using the High Capacity cDNA Reverse Transcription Kit with RNase Inhibitor {Applied Biosystems Inc.} according to the instruction manual. The resultant cDNA was analyzed by real-time PCR method using the Taqman Gene Expression Master Mix {Applied Biosystems, Inc.} according to the instruction manual with the use of the ABI Prism 7900HT sequence detector, and the Ct values <Threshold Cycle> of human profilaggrin, loricrin, involucrin, β-defensin 3 and glyceraldehyde-3-phosphate dehydrogenase <GAPDH> were determined. GAPDH was used as an internal standard. Primers and probes used were purchased from Applied Biosystems, Inc.

### <TaqMan Gene Expression Assays>.

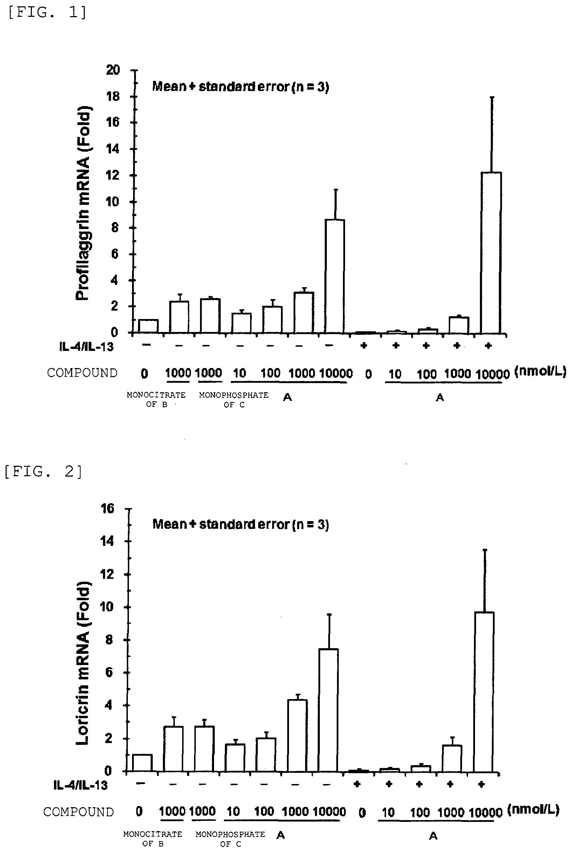

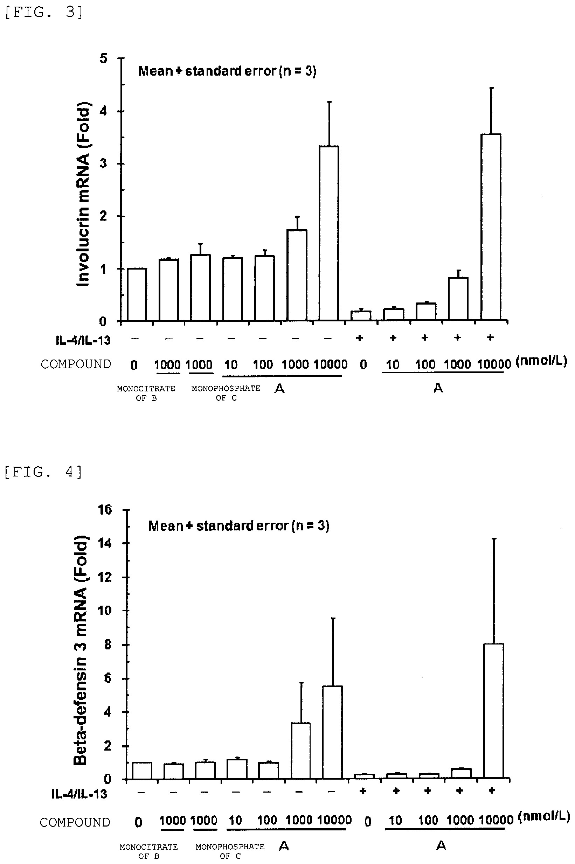

The relative mRNA amount of each of human profilaggrin, loricrin, involucrin and β-defensin 3 was calculated by comparative Ct method. The mRNA amount of the cells to which each test substance had been added was shown in a ratio to the mRNA amount of each gene in the cells to which human IL-4, human IL-13 and a test substance had not been added. The results are shown in Figs. 1 to 4.

### [Example 2]

### Experiment 2. NMF production increasing action by JAK inhibitor in Tape Stripping-treated mouse

Using a Tape Stripping-treated mouse <Non-Patent Literature 9>, the NMF production increasing action by the JAK inhibitor was evaluated. As an experimental animal, an 8 to 10 weeks old female C57BL/6j mouse {SHIMIZU Laboratory Supplies Co., Ltd.} was used.

The operation of sticking a cellophane adhesive tape {NICHIBAN CO., Ltd.} on the inner side of the left auricle of the mouse and peeling the tape off <Tape Stripping> was repeated five times to peel off the cuticle. Each 0.02 mL of acetone <Vehicle> or 0.5% (w/v) test substance was applied to the site having undergone the Tape Stripping once a day until the sixth day after the Tape Stripping. On Day 1 of the Tape Stripping, acetone <Vehicle> or a test substance was applied one hour after the Tape stripping. Compound A or monocitrate of Compound B was used as a test substance.

After one, five and seven days from the Tape Stripping, the total NMF amount of the inner side of the left auricle was determined by an in vivo confocal Raman spectrometer {model 3510, River Diagnostics, Inc.}.

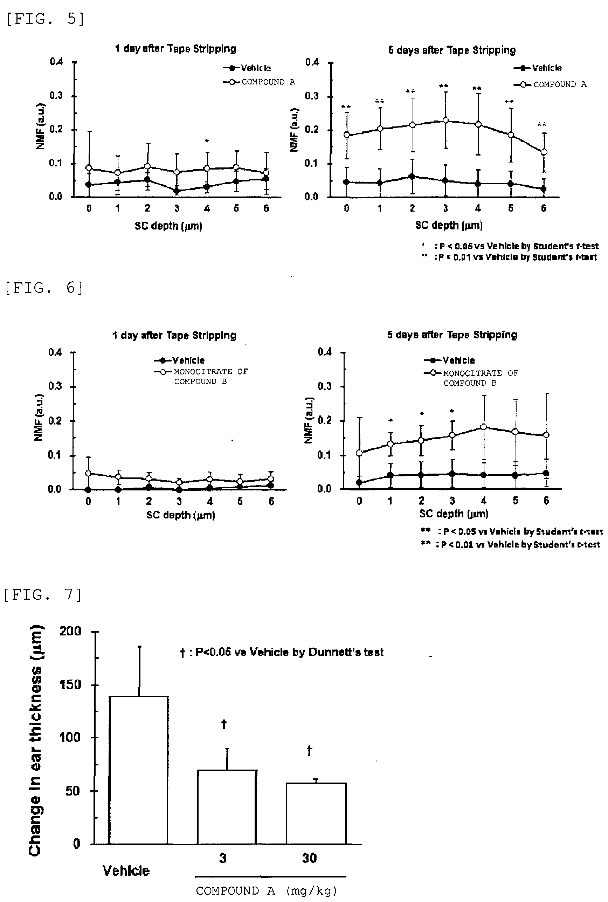

A Raman spectrum in the region of 400 to 1800 cm⁻¹ was measured at an excitation wavelength of 785 nm at intervals of 1 micrometer from the surface of the inner side of the left auricle to the depth of 8 micrometers. A total NMF amount was quantified as an amount per keratin by using Skin Tools 2.0 {River Diagnostics, Inc.}. At each measuring time, the measurement was conducted twice to five times for each individual. An average value of the total NMF amount was calculated for each measurement depth, and was shown as an individual value at each depth. The result of Compound A was shown in Fig. 5, and the result of monocitrate of Compound B was shown in Fig. 6.

### [Example 3]

### Experiment 3. Suppression of ear swelling by JAK inhibitor in contact dermatitis model mouse

Using a contact dermatitis model mouse against DNFB <Non-Patent Literature 10>, the ear swelling suppressing action of the JAK inhibitor was evaluated. As an experimental animal, an 8 weeks old female C57BL/6j mouse {SHIMIZU Laboratory Supplies Co., Ltd.} was used.

An abdominal region of the mouse was shaved, and each 0.025 mL of 0.5% (w/v) DNFB {2,4-Dinitrofluorobenzene, Sigma-Aldrich Corporation} diluted with AOO <mixed solution of acetone and olive oil in 4:1> was applied on the abdominal region <sensitization>. After five days, each 0.01 mL of 0.3% (w/v) DNFB diluted with AOO was applied on the front and back of both the auricles of the mouse <initiation>. For five days from the date of sensitization, 0.5% (w/v) methylcellulose <MC> was administered once a day to the Vehicle group, and 0.3 mg/mL or 3 mg/mL of Compound A suspended in 0.5% (w/v) MC was orally administered to Compound A administration group in a dose of 10 mL/Kg. Each group included three animals. On the days of sensitization and initiation, 0.5% (w/v) MC or Compound A was orally administered one hour before sensitization and initiation.

Before initiation and after 24 hours from initiation, the thickness of each auricle was measured once with a thickness gauge {TECLOCK Corporation}, and an average of the variation from the thickness of each auricle before initiation was taken as an individual value. The result was shown in Fig. 7.

### [Example 4]

### Experiment 4. Increase in protein amount of skin barrier-related protein by JAK inhibitor in three-dimensional culture skin model

As a three-dimensional cultured skin model, TESTSKIN LSE-high {TOYOBO CO., LTD., TMLSE-013A} was used <Non-Patent Literature 11>. According to the instruction manual of the TESTSKIN LSE-high, the Living Skin Equivalent <LSE> tissue on the Transwell was cultured in an assay medium included in the kit at 37°C under 5% CO₂ for 24 hours.

After removing the medium from the LSE tissue culture system, an assay medium containing Compound A in a final concentration of 1000 nmol/L was added in the presence or absence of human IL-4 and human IL-13 {R&D Systems, Inc.} in a final concentration of 20 ng/mL, respectively, and the LSE tissue was cultured at 37°C under 5% CO₂ for three days. The medium was replaced in the same manner as described above, and the LSE tissue was cultured at 37°C under 5% CO₂ for another two days. The skin layer of the LSE tissue was collected in a tube, and 0.1 mL of a 50 mmol/L Tris-HCl (pH 8.0) buffer containing 9 mol/L urea, 2% Triton X-100, protease inhibitor cocktail {Sigma-Aldrich Corporation, P8340} and phosphatase inhibitor cocktail {NACALAI TESQUE, INC., 07575-51} was added, and lysed by ultrasonication. To the tissue lysate, 0.08 mL of laemmli SDS-PAGE sample buffer containing 1.73 mmol/L 2-mercaptoethanol was added, and heated at 95 degrees Celsius for five minutes to thermally denature proteins.

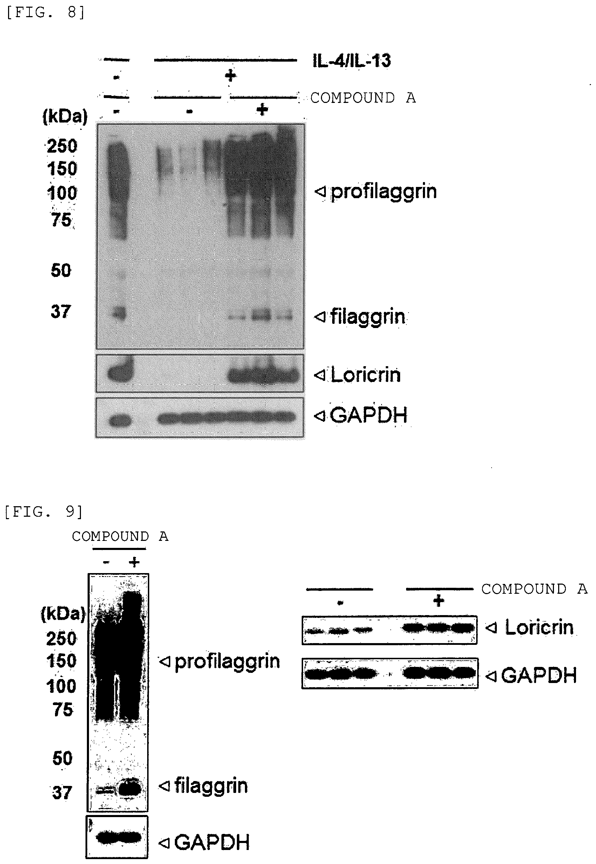

The thermally denatured proteins (0.015 mg/lane) were separated by SDS-polyacrylamide electrophoresis <SDS-PAGE> on 4-12% NuPAGE Bis-Tris gel {Life technologies Corporation}, and filaggrin protein and loricrin protein were detected by Western blotting method. As an internal standard, GAPDH protein was detected. According to ordinary methods, the protein that was separated by SDS-PAGE was blotted on a PVDF membrane {GE Healthcare Ltd. }, and the PVDF membrane was blocked with 5% skim milk {NACALAI TESQUE, INC.}. After incubating the PVDF membrane with a primary antibody <mouse anti-human filaggrin antibody {Santa Cruz Biotechnology, Inc., sc-66192, ×300 dilution}, rabbit anti-human loricrin antibody {Covance Inc., PRB-145P, ×1000 dilution} or rabbit anti-human GAPDH antibody {GeneTex, Inc., GTX100118, ×1000 dilution}>, the membrane was incubated with a secondary antibody labeled with horseradish peroxidase <HRP> <sheep anti-mouse IgG antibody {GE Healthcare Ltd., RPN2124, ×10000 dilution} or goat anti-rabbit IgG antibody {Cell Signaling Technology, Inc., 7074, ×3000 dilution}>. The protein of interest was detected by light exposure to an autoradiography film, Amersham Hyperfilm ECL {GE Healthcare Ltd.} from the PVDF membrane, by using a chemiluminescent substrate {ECL Western Blotting Detection Reagents; GE Healthcare Ltd., RPN2232} according to the instruction manual. The results were shown in Figs. 8 and 9.

### [Example 5]

### Experiment 5. Increase in profilaggrin mRNA amount, increase of NMF production and acceleration of reduction in TEWL by JAK inhibitor in dry skin model mouse

### "Production of dry skin model mouse"

Using a dry skin model mouse <Non-Patent Literature 12>, the effect of improving the skin barrier function by a JAK inhibitor was evaluated. As an experimental animal, an 8 weeks old female C57BL/6JJmsSlc mouse {Japan SLC, Inc.} was used.

Absorbent cotton dipped in a mixed liquid of acetone and diethyl ether (1:1) was put on the surface of the inner side of the right or both auricles of the mouse for 30 seconds, and then absorbent cotton dipped in water was put on the same region for 30 seconds <hereinafter, abbreviated as an A/E/W treatment>. This treatment was conducted twice a day for seven days for each group to produce a dry skin model. The day on which the A/E/W treatment started was taken as Day 0. As a normal control group, a mouse not having undergone the A/E/W treatment was used.

Each 0.02 mL of Compound A having a final concentration of 0.5% (w/v) dissolved in 1% (v/v) DMSO-containing acetone was applied to the region where the A/E/W treatment was conducted directly after the A/E/W treatment once a day for seven days from Day 0 <Compound A administration group>. For the Vehicle group, 0.02 mL of 1% (v/v) DMSO-containing acetone was applied to the region where the A/E/W treatment was conducted directly after the A/E/W treatment once a day for seven days from Day 0.

### "Quantitative real-time PCR"

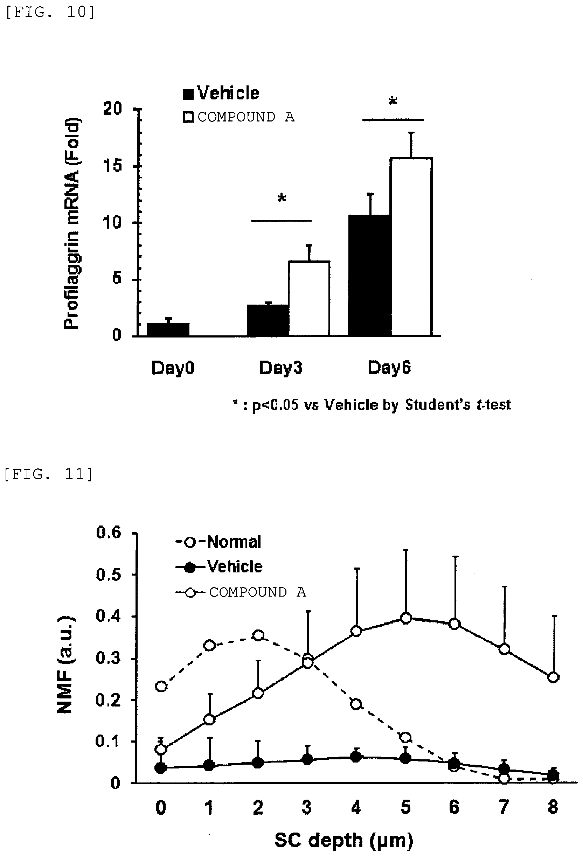

A mouse having undergone the A/E/W treatment on its right auricle was used in the experiment. Directly before the A/E/W treatment on Days 0, 3 and 6, total RNA was extracted from the mouse right auricle <Compound A administration group and Vehicle group; n = 4, respectively> by using an RNeasy mini kit {QIAGEN N.V.} according to the instruction manual, and then digested with DNase I {Takara Bio Inc.}. The total RNA was reverse transcribed into cDNA by using a Prime Script RT reagent kit {Takara Bio Inc. } according to the instruction manual. The cDNA was analyzed by real-time PCR method <intercalator method> using Light Cycler 480 SYBR Green I Master {Roche Diagnostics K.K.} and Light Cycler 480 II {Roche Diagnostics K.K.}, and the Ct values of mouse profilaggrin and GAPDH were respectively determined. GAPDH was used as an internal standard. All the primers used herein were purchased from Greiner Japan. For mouse profilaggrin, 5'-ATG TCC GCT CTC CTG GAA AG-3' was used as a forward primer, and 5'-TGG ATT CTT CAA GAC TGC CTG TA-3' as a reverse primer, and for mouse GAPDH, 5'-GGC CTC ACC CCA TTT GAT GT-3' was used as a forward primer, and 5'-CAT GTT CCA GTA TGA CTC CAC TC-3' as a reverse primer. Measurement was conducted twice for each sample, and an average value thereof was taken as each sample value. A relative mRNA amount of mouse profilaggrin was calculated by comparative Ct method. A mRNA amount of each sample was shown as a ratio to the mRNA amount of mouse profilaggrin on Day 0. The result was shown in Fig. 10.

### "Measurement of NMF"

A mouse having undergone the A/E/W treatment on its both auricles was used in the experiment. On Day 7, the total NMF amount of the stratum corneum of both the auricles of the mouse was determined by an in vivo confocal Raman spectrometer {model 3510, River Diagnostics, Inc. } <Compound A administration group and Vehicle group; n = 3, respectively>.

A Raman spectrum in the region of 400 to 1800 cm⁻¹ was measured at an excitation wavelength of 785 nm at intervals of 1 micrometer from the surface of the inner side of the left auricle to the depth of 8 micrometers. A total NMF amount was quantified as an amount per keratin by using Skin Tools 2.0 {River Diagnostics, Inc.}. For each auricle, the measurement was conducted four times. An average value of the total NMF amount was calculated for each measurement depth, and was shown as an individual value at each depth. As a normal control, a total NMF amount of the auricle stratum corneum of a mouse not having undergone the A/E/W treatment <n=1> was measured. The result was shown in Fig. 11.

### "Measurement of TEWL"

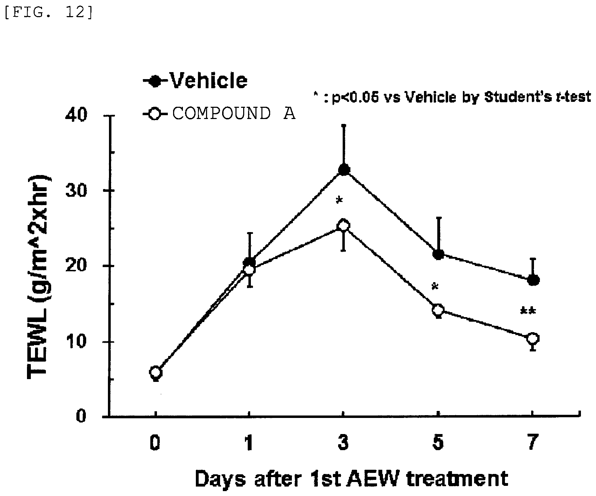

A mouse having undergone the A/E/W treatment on its right auricle was used in the experiment. TEWL in the region where the A/E/W treatment had been conducted was measured directly before the A/E/W treatment on Days 0, 1, 3 and 4, and on Day 7 <Compound A administration group and Vehicle group; n = 4, respectively>. TEWL was measured by using a water loss measuring device VAPOSCAN AS-VT100RS {ASAHIBIOMED} at room temperatures (23°C to 26°C) and a humidity of 40% to 60%. The measurement was conducted twice for each individual, and an average value was taken as each individual value. The result was shown in Fig. 12.

### [INDUSTRIAL APPLICABILITY]

The present invention provides a novel pharmaceutical use of a JAK inhibitor for skin diseases as target diseases.

## Claims

1. An agent for use in the prevention or treatment of a skin disease selected from the group consisting of senile xerosis, asteatosis, ichthyosis vulgaris, Netherton syndrome and type B peeling skin syndrome, comprising a JAK inhibitor as an active ingredient, wherein the JAK inhibitor is a compound represented by the following chemical structural formula: 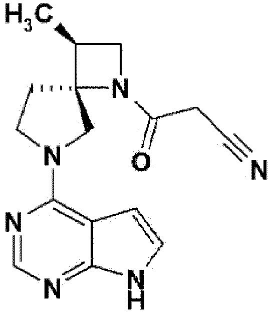 or a pharmaceutically acceptable salt thereof.

2. The agent for use according to claim 1, wherein the skin disease selected in claim 1 is senile xerosis.

3. The agent for use according to claim 1, wherein the skin disease selected in claim 1 is asteatosis.

4. The agent for use according to claim 1, wherein the skin disease selected in claim 1 is ichthyosis vulgaris.

5. The agent for use according to claim 1, wherein the skin disease selected in claim 1 is Netherton syndrome.

6. The agent for use according to claim 1, wherein the skin disease selected in claim 1 is type B peeling skin syndrome.

## Patentansprüche

1. Ein Mittel zur Verwendung bei der Vorbeugung oder Behandlung einer Hauterkrankung, ausgewählt aus der Gruppe bestehend aus seniler Xerose, Asteatose, Ichthyosis vulgaris, Netherton-Syndrom und Peeling Skin-Syndrom Typ B, umfassend einen JAK-Inhibitor als Wirkstoff, wobei der JAK-Inhibitor eine Verbindung, dargestellt durch die folgende chemische Strukturformel: 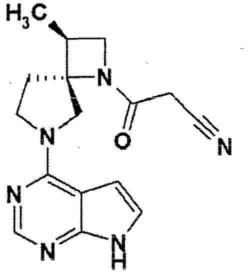 oder ein pharmazeutisch verträgliches Salz davon ist.

2. Das Mittel zur Verwendung nach Anspruch 1, wobei die in Anspruch 1 ausgewählte Hauterkrankung senile Xerose ist.

3. Das Mittel zur Verwendung nach Anspruch 1, wobei die in Anspruch 1 ausgewählte Hauterkrankung Asteatose ist.

4. Das Mittel zur Verwendung nach Anspruch 1, wobei die in Anspruch 1 ausgewählte Hauterkrankung Ichthyosis vulgaris ist.

5. Das Mittel zur Verwendung nach Anspruch 1, wobei die in Anspruch 1 ausgewählte Hauterkrankung Netherton-Syndrom ist.

6. Das Mittel zur Verwendung nach Anspruch 1, wobei die in Anspruch 1 ausgewählte Hauterkrankung Peeling Skin-Syndrom Typ B ist.

## Revendications

1. Agent destiné à être utilisé dans la prévention ou le traitement d'une maladie de la peau choisie dans le groupe consistant en la xérose sénile, l'astéatose, l'ichtyose vulgaire, le syndrome de Netherton et le syndrome de desquamation cutanée de type B, comprenant un inhibiteur de JAK comme ingrédient actif, où l'inhibiteur de JAK est un composé représenté par la formule structurale chimique suivante: 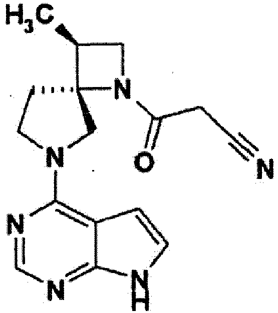 ou sel pharmaceutiquement acceptable de celui-ci.

2. Agent destiné à être utilisé selon la revendication 1, où la maladie de la peau choisie dans la revendication 1 est la xérose sénile.

3. Agent destiné à être utilisé selon la revendication 1, où la maladie de la peau choisie dans la revendication 1 est l'astéatose.

4. Agent destiné à être utilisé selon la revendication 1, où la maladie de la peau choisie dans la revendication 1 est l'ichtyose vulgaire.

5. Agent destiné à être utilisé selon la revendication 1, où la maladie de la peau choisie dans la revendication 1 est le syndrome de Netherton.

6. Agent destiné à être utilisé selon la revendication 1, où la maladie de la peau choisie dans la revendication 1 est le syndrome de desquamation cutanée de type B.


### SEQUENCE LISTING

<110> JAPAN TOBACCO INC.;KYOTO University
<120> Agent for improvement of skin barrier function
<130> JF1041JP
<150> JP 2013-092378
   <151> 2013-04-25
<160> 4
<210> 1
   <211> 20
   <212> DNA
   <213> Mus musculus
<400> 1
   atgtccgctctcctggaaag
<210> 2
   <211> 23
   <212> DNA
   <213> Mus musculus
<400> 2
   tggattcttcaagactgcctgta
<210> 3
   <211> 20
   <212> DNA
   <213> Mus musculus
<400> 3
   ggcctcaccccatttgatgt
<210> 4
   <211> 23
   <212> DNA
   <213> Mus musculus
<400> 4
   catgttccagtatgactccactc